(19) Europäisches Patentamt
European Patent Office
Office européen des brevets

(11) EP 1 001 978 B1

(12) EUROPEAN PATENT SPECIFICATION

(45) Date of publication and mention of the grant of the patent:
**30.03.2005 Bulletin 2005/13**

(21) Application number: **98930927.3**

(22) Date of filing: **23.06.1998**

(51) Int Cl.[7]: **C07K 7/14**, C07K 14/00, A61K 47/48, C12N 15/16

(86) International application number:
**PCT/GB1998/001833**

(87) International publication number:
**WO 1998/058952 (30.12.1998 Gazette 1998/52)**

# (54) ANGIOTENSIN DERIVATIVES

ANGIOTENSINDERIVATE

DERIVES D'ANGIOTENSINE

(84) Designated Contracting States:
**AT BE CH CY DE DK ES FI FR GB GR IE IT LI LU MC NL PT SE**

(30) Priority: **24.06.1997 GB 9713361**
**23.04.1998 GB 9808696**

(43) Date of publication of application:
**24.05.2000 Bulletin 2000/21**

(60) Divisional application:
**04016314.9 / 1 486 505**

(73) Proprietor: **Proteus Molecular Design Limited**
**Macclesfield, Cheshire SK11 0JL (GB)**

(72) Inventors:
- **GLOVER, James, Francis**
  **Congleton, Cheshire CW12 3DS (GB)**
- **RUSHTON, Arthur**
  **Wilmslow, Cheshire SK9 2BE (GB)**
- **MORGAN, Phillip, John**
  **Congleton, Cheshire CW12 3RP (GB)**
- **YOUNG, Stephen, Clinton**
  **Stockport, Cheshire SK4 4LD (GB)**

(74) Representative: **Cockbain, Julian, Dr.**
**Frank B. Dehn & Co.,**
**European Patent Attorneys,**
**179 Queen Victoria Street**
**London EC4V 4EL (GB)**

(56) References cited:
**WO-A-93/08842** **US-A- 4 384 995**
**US-A- 5 229 490**

- **J.M PEETERS ET AL.: "Comparison of four bifunctional reagents for coupling peptides to proteins..." J. IMMUNOLOGICAL METHODS, vol. 120, 1989, pages 133-143, XP002080173**
- **K.Y. KUMAGAYE ET AL.: "Suppression of a side reaction associated with Nim-benzyloxymethyl group during synthesis of peptides containing cysteinyl residue at the N-terminus" PEPTIDE RESEARCH, vol. 4, no. 2, 1991, pages 84-87, XP002080174**
- **KAWABE H ET AL: "CHARACTERIZATION OF RECEPTORS FOR ANGIOTENSIN-INDUCED DRINKING AND BLOOD PRESSURE RESPONSES IN CONSCIUS RATS USING ANGIOTENSIN ANALOGSEXTENDED AT THE N-TERMINAL" NEUROENDOCRINOLOGY, vol. 42, 1986, pages 289-295, XP002058941**
- **F.S. TJOENG ET AL.: "Multiple peptide synthesis using a single support (MPS3)" INTERNATIONAL JOURNAL OF PEPTIDE AND PROTEIN RESEARCH, vol. 35, 1990, pages 141-146, XP002080175 COPENHAGEN DK**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 001 978 B1

## Description

The present invention relates to derivatives of the mammalian peptide hormone angiotensin I and to immunotherapeutic uses of these in particular for the therapy and prophylaxis of conditions associated with the renin activated angiotensin system.

Angiotensin peptides are involved in controlling arterial pressure in mammals. They are produced in several forms in the body as a result of a biochemical cascade known as the renin-angiotensin system (RAS), initiated by renin produced as a result of a fall in arterial pressure. In the RAS, represented schematically below, renin is released by the kidneys from stored pro-renin following a fall in arterial blood pressure, and acts enzymatically upon angiotensinogen to produce angiotensin I which is a decapeptide having the sequence Asp-Arg-Val-Tyr-Ile-His-Pro-Phe-His-Leu. Two amino acids from the C-terminus of angiotensin I are rapidly cleaved, by angiotensin converting enzyme (ACE), present in the endothelium of the lungs, generally within 1-2 seconds, to produce the octapeptide angiotensin II, having the sequence Asp-Arg-Val-Tyr-Ile-His-Pro-Phe.

```
Angiotensinogen ----------> angiotensin I-----> angiotensin II
                     ↑                    ↑
                   renin      angiotensin converting enzyme (ACE)
```

Angiotensin I is very short lived within the body and has mild vasoconstrictor activity. Alone therefore it has insignificant effect on the circulatory system. Angiotensin II, however, is a vasoactive peptide which has a profound effect on the circulatory system, as well as on the endocrine system. Elevated levels of RAS-activated angiotensin II cause vasoconstriction and renal retention of salt and water, both of which contribute to increased arterial pressure (hypertension) which can lead to cardiovascular damage. Angiotensin II has been implicated in a number of other disease states, including congestive heart failure. Hypertension is a major risk factor for heart attacks and strokes and congestive heart failure is the disease with the highest mortality within a few years of onset. There is a need for effective therapies for combatting these and other diseases associated with the renin-angiotensin system.

The use of angiotensin I, or rather the desaspartate equivalent in treatment of cardiac problems is described in WO 96/37213.

Current treatment for these diseases includes intervention in the RAS system using small organic molecules. One approach attempts to inhibit ACE with inhibitors such as lisinopril, captopril and enalapril, agents which are now established in management of hypertension. These drugs have not however been entirely successful. It seems that inhibition of ACE is only partial. Furthermore, because ACE lacks substrate specificity, biotransformation of other metabolically active peptides, including bradykinin may also be inhibited, which is undesirable. In addition, these drugs need to be taken on a regular basis, often for long periods, such as for the majority of adult life. A major drawback, however, of these drugs is their undesirable side effects, including dry cough and a first dose hypotensive effect with dizziness and possible fainting. Since anti-hypertensive therapies invariably need to be taken long term, e.g. for up to 30 years and sometimes even longer, these adverse side effects can result in loss of patient compliance, particularly in the absence of short term clinical benefit in a mainly asymptomatic condition, severely limiting the usefulness of this therapeutic approach.

A more recent therapeutic approach involves drugs which are angiotensin receptor antagonists which are intended to block the activity of angiotensin II. Examples include losartan and valsartan. The agents which have been developed to date appear to be specific for only the $AT_1$ angiotensin receptor; they therefore block the dominant vasoconstrictor effects of angiotensin II, and are better tolerated but do not affect other actions of the angiotensin hormones. Experience with $AT_1$ receptor antagonists indicates that whilst they may be of comparable effectiveness to ACE inhibitors poor patient compliance remains a problem. There is accordingly a need for improved therapies of diseases associated with the RAS.

A potential approach in treating or preventing diseases or disorders associated with the activity of a hormone is to neutralise the effects of the hormone within the patient by immunotherapy i.e. by immunising the patient against the hormone such that the activity of the hormone is neutralised by specific anti-hormone antibodies. Such antibodies may be exogenously administered in passive immunisation or they may be generated *in situ* by active immunisation using an immunogen based on the hormone.

We have now developed new derivatives of angiotensin which are potent immunogens and which can be used in an immunotherapeutic approach to combat conditions associated with elevated levels of angiotensin II produced by the RAS.

In particular, derivatives of angiotensin have been developed in which one or more angiotensin peptides are coupled to a binder moiety, e.g. a peptide sequence, which facilitates attachment of the angiotensin peptide to an

immunological carrier such as a protein or polypeptide to form an immunogenic conjugate capable in an immunised host of inducing antibodies which bind to angiotensin and neutralise its effects. These induced antibodies include those which may also bind to the precursor form, angiotensinogen and in this way, cleavage by renin to angiotensin I is prevented, thereby providing an additional blockade of the system. This may be particularly relevant to reducing the effects of modulation of the negative feedback effects of Angiotensin II on renin production and release of Angiotensin I.

The production of peptide conjugates is known: thus WO 93/08842 describes hemoglobin conjugates and Peeters et al. in J. Immunol. Methods 120: 133-143 (1989) describes production of immunogenic peptide-carrier conjugates. In one aspect, the present invention thus provides the use of an immunogenic angiotensin conjugate comprising at least one angiotensin I peptide coupled via a peptide carrier-binding moiety to a carrier for the manufacture of a medicament for use in combatting diseases associated with the renin angiotensin system.

Such diseases include congestive heart failure and hypertension such as systemic hypertension and other diseases in which the renin-angiotensin system contributes to the pathophysiology thereof, as well as diseases where the renin-angiotensin system has elevated levels of activity.

These angiotensin conjugates may be used to immunise a patient against the hormone angiotensin II and/or its polypeptide precursor angiotensin I and/or angiotensinogen such that the activity of the hormone is neutralised by specific anti-hormone or antipolypeptide antibodies.

The angiotensin I peptide conveniently comprises a decapeptide of formula Asp-Arg-Val-Tyr-Ile-His-Pro-Phe-His-Leu.

The peptide carrier-binding moiety serves as a means by which the angiotensin peptide may be attached to an immunological carrier, which will generally be a protein or polypeptide, and thus preferably contains an amino acid residue having a reactive side chain, via which the angiotensin derivative may readily be coupled to the carrier using standard coupling techniques. Advantageously such a side chain may contain a free hydroxyl, carboxyl or thiol group. Such an amino acid may thus conveniently be a cysteine, tyrosine, aspartic acid or glutamic acid residue or a derivative thereof such as N-acetyl cysteine.

The angiotensin carrier-binding moieties have been shown to have improved coupling to an immunological carrier for inducing antibodies which can be used immunotherapeutically and these angiotensin carrier-binding moieties have advantages in this regard over the native peptide.

The carrier-binding moiety may take the form of a peptide extension at the N- or C-terminus of an angiotensin peptide, or a peptide pendant from or disposed within a chain segment between two or more angiotensin moieties.

In a preferred embodiment of the present invention the conjugates are conjugates of a carrier and a compound of Formulae

N-acetyl-Cys-Ala-Angiotensin I

N-acetyl-Cys-$(Ala)_4$-Angiotensin I

N-acetyl-Cys$(Gly)_6$-Angiotensin I

N-acetyl-Cys-Gly-Ala-Gly-Ala-Angiotensin I

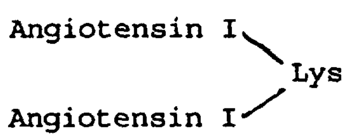

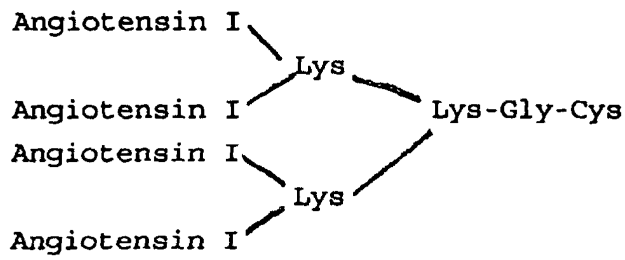

(A)-Gly Cys

N-acetyl-Cys-(A)

N-acetyl-Cys-Gly-(A)

wherein A is angiotensin I.

Although the peptide analogues of the invention when examined by computer-aided energy minimisation modelling are generally considered too small to be optimally immunogenic alone, it has been found that when coupled via the carrier-binding moiety to a carrier, these peptide analogues elicit a strong protective immune response. They are thus eminently suitable for use in immunotherapy against RAS-associated conditions. Without wishing to be bound by theory, it is believed that coupling of the peptides to a carrier by means of the carrier-binding moiety results in the analogues having substantially the same conformation as that of the native angiotensin peptides.

The new derivatives according to the invention may be generated using a number of standard techniques including, for peptides, the Merrifield solid phase method in which amino acids are added stepwise to a growing polypeptide linked to a solid matrix as described in R.B. Merrifield, Fed. Proc. Amer. Soc. Biol. (1962). 21, 412 and R.B. Merrifield, Jour. Amer. Chem. Soc. (1963), 85, 2149 and conventional FMOC chemistry. If desired, reactive side chain groups of the amino acids in the growing chain may be protected during the chain synthesis. Branched structures may be prepared by similar techniques.

Where the new derivatives are linear peptides these may also be prepared by recombinant DNA expression using techniques known in the art e.g. as described, for example, by Sambrook et al., in Molecular Cloning: A Laboratory Manual, Second Edition, 1989.

The angiotensin:carrier binding moiety, as is the case for other small molecules, may be of insufficient size to stimulate antibody formation alone and may thus need to be conjugated to a macromolecular carrier in order to stimulate antibody production and a protective immune response.

Coupling to the carrier may be by methods known in the art for example by treatment with heterobifunctional linking agents. Where coupling is via a terminal cysteine (or N-acetyl cysteine), the linking agent may be m-Maleimidobenzoyl-N-hydroxysulphosuccinamide ester; in which case maleimide modifies one or more lysine side chains in the peptide carrier, and a thioether bond forms at the terminal cysteine residue. Other coupling reagents known in the art, eg carbodiimide coupling, may also be used.

Any carrier known in the art for such purposes may be used, including the purified protein derivative of tuberculin, tetanus toxoid, diphtheria toxoid, keyhole limpet haemocyanin or derivatives thereof.

Where the angiotensin:carrier binding moiety is a linear peptide and the carrier is a protein or polypeptide, the entire peptide conjugate may also be made by recombinant DNA methods wherein a nucleic acid molecule encoding the conjugated molecule is expressed in an appropriate host cell.

The angiotensin conjugates of the invention may be used in an immunotherapeutic approach to combatting diseases associated with normal or elevated levels of RAS activity and/or angiotensin peptides, and represents an advantageous method compared to currently available methods. Patient compliance should be increased in that less frequent dosing than is the case with current therapies is involved, and undesirable side effects are avoided.

Viewed from a still yet further aspect, the invention provides the use of an angiotensin conjugate according to the invention for the modulation of blood pressure.

The angiotensin conjugate according to the invention may be administered by all conventional methods including parenterally (e.g. intraperitoneally, subcutaneously, intramuscularly, intradermally for example in the form of inert particles such as gold pellets or beads to which the derivative is adsorbed which may be accelerated at speeds sufficient to enable them to penetrate the skin of a subject, or intravenously), topically (e.g. as a cream to the skin),

intra-articularly, mucosally (e.g. orally, nasally, vaginally, rectally and via the intra-ocular route) or by intrapulmonary delivery for example by means of devices designed to deliver the agents directly into the lungs and bronchial system such as inhaling devices and nebulisers, and formulated according to conventional methods of pharmacy optionally with one or more pharmaceutically acceptable carriers or excipients, such as for example those described in Remingtons Pharmaceutical Sciences, ed. Gennaro, Mack Publishing Company, Pennsylvania, USA (1990).

Such compositions are conveniently formulated in unit dosage form e.g. for mucosal, parenteral or oral administration.

Actual treatment regimes or prophylactic regimes, formulations and dosages will depend to a large extent upon the individual patient and may be devised by the medical practitioner based on the individual circumstances.

The type of formulation will be appropriate to the route of administration. For example, parenteral administration by subcutaneous or intramuscular injection may be with a sterile aqueous suspension of the conjugated analogue in PBS, saline or water for injection, optionally together with one or more immunological adjuvants e.g. aluminium hydroxide, saponin, quil A, muramyl dipeptide, mineral or vegetable oils, vesicle-based adjuvants, non-ionic block copolymers, or DEAE dextran. Additional components such as preservatives may be used.

The dosage for injection may be in the range 1-100 μg peptide equivalent and the frequency of administration may be upwards of from once every three or six months, to once every year or once every five years.

For oral administration, the conjugated derivatives may be formulated as tablets, liquid, capsules etc. Dosages range from 1 to 1000 μg peptide equivalent with dosing occurring at intervals dependent on bioavailability of product.

The invention will now be described in further detail in the following non-limiting Examples, with reference to the drawings in which:

Figure 1 is a graph showing antibody titres +/sem, n=6 (dilution corresponding to 0.1 increase in OD) against time (sample day);

A = Control
B = Derivative 3 of Example 2
C = Derivative 1 of Example 2
D = Derivative 4 of Example 2
E = Derivative 2 of Example 2.

Figure 2 is a graph showing peak change in blood pressure following administration of A1 in control rats and in rats immunised with a conjugate of an analogue of A1 in groups C and J of Example 4.

Figure 3 shows recordings of mean blood pressure changes in response to Al in animals of groups A and C of Example 4.

Figures 4, 5 and 6 are bar charts showing antibody titres measured in terms of $A^{450}$ in an ELISA assay using in the assay in Figure 4 angiotension I, in Figure 5 angiotensin II and in Figure 6 angiotensinogen, the ELISAs showing the binding of partially purified rat antisera raised against vaccines containing analogues of angiotensin hormones.

Figures 7 and 8 are graphs showing antibody titres against time (sample day) for the following derivatives

N-acetyl-Cys-$(Ala)_4$-Angiotensin I
N-acetyl-Cys$(Gly)_6$-Angiotensin I

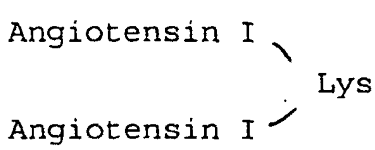

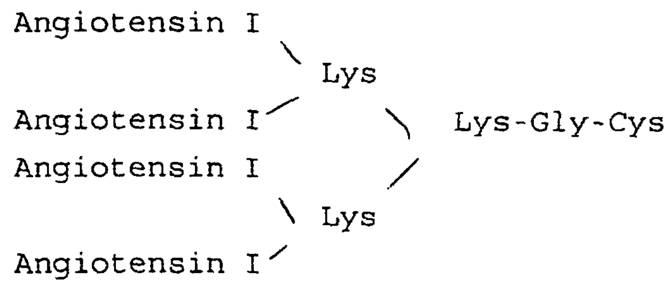


**Example 1:** Peptide generation.

Peptides were synthesised by the Fmoc strategy of solid phase peptide synthesis on a Protein Technologies, Symphony Peptide Synthesiser. The resin used was Tentagel S-NH2 with a Rink Amide linker. The side chain protecting groups of the Fmoc amino acids used were Trt for Cys His, Asn and Gln, tBu for Tyr Thr, Asp, Glu and Ser; Boc for Lys and the indole N of Trp, Pmc for Arg. Activation of the carboxyl groups was achieved using, TBTU/HOBt/DIPEA, all couplings were carried out in DMF. Deprotection of the Fmoc groups was achieved with 20% Piperidine in DMF. Cleavage of the peptides from the resin was carried out with 5%Anisole/5%Thioanisole/5%EDT/3%Water/2%TES in TFA for 1 hour. The peptides were purified by RP-HPLC using a 40mm x 210mm Deltapak C18 radial compression column on a Waters Deltaprep 4000 and characterised by MALDI-TOF on a Kratos Maldi 3 and by AAA.

For dendrimers Fmoc Lys(Fmoc)-OH is attached by the methods above and gives both α and ε amino groups free for peptide elongation. Quantities of Fmoc amino acids used have to be increased accordingly.

Rink Amide Linker = p-[(R,S--[1-(9H-Fluoren-9-yl)-methoxyformamido]-2,4-dimethoxybenzyl]-phenoxyacetic acid

Fmoc =9-Fluorenylmethoxycarbonyl
Trt = Trityl, Triphenylmethyl
tBu = tertiary butyl
Boc = tertiary butyloxycarbonyl
Pmc = 2,2,5,7,8-Pentamethylchroman-6-sulphonyl
TBTU = 2-(1H-Benzotriazole-1-yl)-1,1,3,3-tetramethyluronium tetrafluoroborate
HOBt = N-Hydroxybenzotriazole
DIPEA = Diisopropylethylamine
DMF = N,N Dimethylformamide
EDT = Ethanedithiol
TES = Triethylsilane
TFA = Trifluoroacetic acid
RP-HPLC = Reverse phase high performance liquid chromatography
MALDI-TOF = Matrix assisted laser desorption ionisation - time of flight
AAA = Amino acid analysis
Fmoc-Lys(Fmoc)-OH = α, ε di-9-fluorenylmethoxycarbonyl lysine

The following peptides were synthesized in this manner:

| | |
|---|---|
| **(1) Angiotensin I-gly-cys** | **Asp-Arg-Val-Tyr-Ile-His-Pro-Phe-His-Leu Gly-Cys** |
| **(2) Angiotensin II-gly-cys** | **Asp-Arg-Val-Tyr-Ile-His-Pro-Phe-Gly-Cys** |
| **(3) N-acetyl-Cys-Gly-Angiotensin I** | **N-acetyl-Cys-Gly-Asp-Arg-Val-Tyr-Ile-His-Pro-Phe-His-Leu** |
| **(4) N-acetyl-Cys-Gly-Angiotensin II** | **N-acetyl-Cys-Gly-Asp-Arg-Val-Tyr-Ile-His-Pro-Phe** |

(2) and (4) are comparative Examples

**Example 2:** Conjugation procedure.

To tetanus toxoid solution in phosphate buffered saline (PBS), a 60 molar excess of S-MBS, m-Maleimidobenzoyl-N-hydroxysulphosuccinimide ester is added and stirred for 2 hours at 4°C in a sealed vial.
Excess S-MBS crosslinker is removed by chromatography (gel exclusion, PD-10, G-25 sephadex column) in PBS. The activated tetanus toxoid peak is collected, assayed for free maleimido groups and used as below.
The resulting carrier protein solution is purged with $N_2$, and a 12 molar excess of angiotensin derivative peptide added. The resulting solution is stirred for 4 hours at 20°C in a sealed container.
The conjugate is purified from free peptide by gel exclusion chromatography as above. A final assay for loss of free maleimido groups is performed on a sample of the mixture to prove that all available sites are conjugated.
The final conjugate is diluted to a working concentration and formulated as desired.
The structure of S-MBS crosslinked conjugate with a linear C-terminal extended angiotensin peptide derivative (eg. derivatives (1) and (2) of Example 1) is shown below:

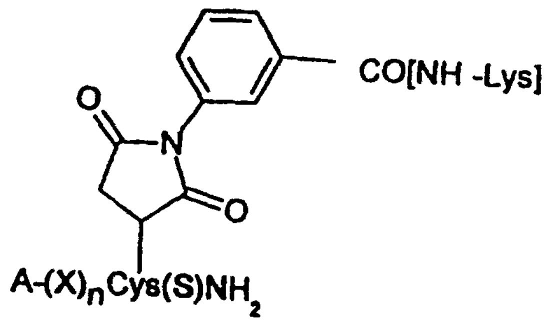

Following this procedure, angiotensin derivatives (1) to (4) of Example 1 were conjugated individually to aliquots of tetanus toxoid.

**Example 3:** Immunisation Studies.

The four angiotensin derivatives of Example 1, conjugated individually to aliquots of tetanus toxoid as described in Example 2, were formulated by adsorption to 0.4% (w/v) aluminium hydroxide gel (Alhydrogel, Superfos s/a, Denmark) in a normal saline (0.9% (w/v)) vehicle.
All conjugates were used as 10µg/ml peptide equivalent solution.
Male Sprague-Dawley rats were used in 5 treatment groups, with 6 rats per group.
The treatment groups received:

| | |
|---|---|
| Vehicle, sterile saline | 0.5 ml/rat |
| N-acetyl-cys-gly-Angiotensin I derivative immunotherapeutic | 5 µg peptide equivalent/ rat in 0.5ml vehicle |
| Angiotensin I-gly-cys derivative immunotherapeutic | 5 µg peptide equivalent/ rat in 0.5 ml vehicle |
| N-acetyl-cys-gly-Angiotensin II derivative immunotherapeutic | 5 µg peptide equivalent/ rat in 0.5 ml vehicle |
| Angiotensin II-gly-cys derivative immunotherapeutic | 5 µg peptide equivalent/ rat |

The route used was subcutaneous and each rat received 3 separate doses of the specified test article during the course of the study.
The bodyweight of each rat is recorded once a week throughout the experimental procedure.

**Experimental procedure**

In this initial investigation the core temperature of each rat was recorded, as part of the general physiological monitoring of the animals.
On day 1 and subsequently on days 22 and 43, the rats received a single subcutaneous dose of the vehicle, or the test articles. Twenty four hours following each administration (days 2, 23 and 44) and on further days specified

in table 1 a venous blood sample (0.5 ml) was subsequently taken while the rat was restrained.

Each sample of venous blood was collected in a glass tube, cooled on ice and allowed to clot, then centrifuged to yield serum within 45 minutes of sampling. Serum samples were frozen at approximately -20°C as soon as possible.

Table 1:

| **Time Schedule of Study Procedures** | | | |
|---|---|---|---|
| **Week** | **Day** | **Treatment** | **Blood Sample** |
| **1** | 1 | Test articles, vehicle | |
| | 2 | No dosing | + |
| **2** | 9 | No dosing | + |
| | 16 | No dosing | + |
| **3** | 22 | Test articles, vehicle | |
| | 23 | No dosing | + |
| **4** | 30 | No dosing | + |
| **5** | 37 | No dosing | + |
| **6** | 43 | Test articles, vehicle | |
| | 44 | No dosing | + |
| **7** | 51 | No dosing | + |
| **8** | 58 | No dosing | + |
| **9** | 65 | No dosing | + |
| **10** | 72 | No dosing | + |
| **11** | 79 | No dosing | + |
| **12** | 86 | No dosing | + |

Each serum sample was assayed for the generation by the treatment of an antibody response by titration of anti-angiotensin peptide-antibodies present in the sera by Enzyme Linked ImmunoSorbant Assay (ELISA).

This assay was performed as follows:

Coat the 96 well uniwell microtitre plates with 50 µl detection substrate e.g. Angiotensin II-Gly-Cys-BSA (10 µg peptide equivalent/well) for 1 hour at room temperature. At the same time place 50 µl PBS into separate wells to act as a substrate blank.

Wash the plates 3 times with 200 µl Phosphate Buffered Saline (PBS)/0.1% Tween 20.

Add 200 µl/well of 3% (w/v) milk powder (Marvel) in PBS and leave for 1 hour at room temperature to block nonspecific antibody binding.

Wash the plates 3 times with 200 µl PBS/ 0.1% Tween 20.

Dilute the serum samples to a suitable dilution with PBS. Typical dilutions would be as follows:

i) 1/100 - 5 µl rats sera + 495 µl PBS
ii) 1/1000 - 20 µl (i) + 180 µl PBS
iii) 1/2000 - 10 µl (i) + 190 µl PBS
iv) 1/5000 - 4 µl (i) + 196 µl PBS

Load the appropriate diluted sera (50 µl) to appropriate wells and incubate at 20°C for 1 hour to permit substrate:antibody binding.

Wash the plates 3 times with 200 µl PBS/0.1% Tween 20.

Dilute rabbit anti-rat IgG peroxidase conjugate 1:5000 in PBS i.e. 1 µl IgG peroxidase + 5 mls PBS. This binds to the rat serum antibody and allows antibody detection.

Add 50 µl of the diluted IgG peroxidase to the appropriate wells and leave for 45 minutes at room temperature.

Wash the plates 3 times with 200 µl PBS.

250 µl aliquot of the perodidase substrate $3,3^1,5,5^1$ ,- tetra methyl benzidine (TMB) to 25 mls 0.1M sodium

acetate buffer pH5.5 with 4 µl 30% hydrogen peroxide.

Add 100 µl of the prepared TMB substrate to the appropriate wells, including the blank wells. A colour producing reaction occurs where antibody/ substrate binding has occurred. Leave for 15 minutes at room temperature, then terminate the reaction with 50 µl 10% sulphuric acid added to each well.

The plate was read for absorbance of light at 405 nm generated by the reaction of the peroxidase enzyme on the TMB substrate and is proportional to the amount of primary (anti-angiotensin) antibody bond. Results for the 4 sample conjugate formulations of derivatives (1) to (4) of Example 1 are shown in Figure 1.

Figure 1 shows a time course of mean antibody titre (+/-Sem, n=6) on the y axis at different sample times, measured in days on the x axis. The titre is the SAS estimated dilution of serum required for a 0.1 OD change from baseline levels in the ELISA assay.

The changes in antibody levels against angiotensin peptides can be seen over time, and are summarised below:

| Immunogen | Peak titre | Day | Terminal titre (Day 86) |
|---|---|---|---|
| (B) N-acetyl-cys-gly-Angiotensin I | 12,218 ± 3576 | 86 | As peak |
| (C) Angiotensin I-gly-cys | 9,535 ± 4423 | 30 | 5068 ± 2038 |
| (D) N-acetyl-cys-gly-Angiotensin II | 15,726 ± 8271 | 30 | 10,239 ± 6544 |
| (E) Anixiotensin II-gly-cys | 5090 ± 2965 | 37 | 2011 ± 1250 |
| (A) is the control; (D) and (E) are comparative | | | |

In parallel with the antibody titre data, all animals were examined for gross physiological changes in body temperature, weight and general appearance, as an overall assessment of toxic or harmful effects.

No adverse effects were recorded on any of the 4 angiotensin immunoconjugate treatment groups, showing that the treatments are effective in generating anti-angiotensin antibodies, without harmful physiological effects in the animals.

**Example 4:** Effects of active immunisation against angiotensin peptides on the pressor effects of exogenous angiotensin I (AI) in conscious rats

In this experiment to demonstrate the potential of active immunisation with angiotensin analogues, certain analogues of angiotensin I (AI) and angiotensin II (AII) were conjugated to carrier proteins which are good immunogens. These immunoconjugates were adjuvanted and shown in immunised rats to generate a strong anti-angiotensin immune response.

The immunised rats were examined with regard to inhibition of the pressor response to exogenous AI.

**Materials and Methods**

**Angiotensin immunotherapeutic vaccine preparation**

The angiotensin analogues used in this study were:

AI analogue is: N-acetyl-cysteine-glycine-angiotensin I
AII analogue is: N-acetyl-cysteine-glycine-angiotensin II (comparative)

The analogues of AI and AII were prepared using a Symphony peptide synthesiser (Anachem).

The conjugation carrier proteins, tetanus toxoid (TT) (Chiron Behring, GmbH), keyhole limpet haemocyanin (KLH) (Biosyn, GmbH) and non toxic recombinant diphtheria toxin (DT) (Chiron Behring, GmbH), were activated using a suitable bivalent linker. The 'activated' carrier protein was separated from the excess cross-linker reagent by size exclusion chromatography.

The following conjugates were made

| Sample Group | Conjugate |
|---|---|
| A | Saline control |

(continued)

| Sample Group | Conjugate |
|---|---|
| B* | AII analogue, TT carrier protein |
| C | AI analogue, TT carrier protein |
| D* | AII analogue, DT carrier protein |
| E* | AII analogue, KLH carrier protein |
| F* | AII analogue, TT carrier protein |
| G | equal mix of AI and AII analogues TT carrier protein |
| H* | AII analogue, TT carrier protein |
| J | AI analogue, TT carrier protein |
| K* | AII analogue, TT carrier protein |
| L | AI analogue, TT carrier protein |

* - B, D, E, F, H and K are comparative

Key: AI/AII Peptide analogues of angiotensin hormones

TT Tetanus toxoid

DT non-toxic recombinant Diphtheria toxin

KLH Keyhole Limpet Haemocyanin

An excess of the AI and/or AII analogues was mixed with the activated carrier proteins and allowed to react, after which AI/AII-carrier protein conjugates were separated from the remaining free analogue by size exclusion chromatography.

The conjugates were sterilised by filtration through a 0.2 μm filter (Millipore) and formulated with adjuvant and saline vehicle to yield the appropriate vaccine for administration.

Alhydrogel® (Superfos S.A.) was the chosen aluminium hydroxide gel adjuvant for this study and 0.9% saline (Flowfusor®, Fresenius) the vaccine vehicle.

Table 2 shows the conjugate formulations administered to each of the treatment groups. The conjugates were formulated with aluminium hydroxide adjuvant, other than the conjugate of Group F which was formulated with DEAE (diethylaminoethyl)-dextran adjuvant.

**Immunisation and AI Challenge**

Male, Sprague Dawley rats (initially 200-250 g: Harlan Olac: n=6 for all groups) were injected (0.5 ml, sc.) with saline or immunotherapeutic vaccines on the days specified in Table 2.

On day 61, under sodium methohexitone anaesthesia (40-60 mg $kg^{-1}$ i.p., supplemented as required), catheters were implanted in the distal abdominal aorta (via the ventral caudal artery) and right jugular vein. The following day, conscious rats were given increasing i.v. bolus (0.1 ml) doses of AI (3-60 pmol $rat^{-1}$), while mean systemic arterial blood pressure and heart rate were recorded. At the end of the experiment animals were given i.v. sodium pentobarbitone (100 mg) and a blood sample was taken by cardiac puncture for the measurement of anti-angiotensin antibodies by ELISA.

**Table 2**
**Treatment regime, formulations, doses, injection frequency and experimental regimes for study**

| | | | Injections | | | | | Catheters | Challenge (AI) |
|---|---|---|---|---|---|---|---|---|---|
| | | Days | 0 | 14 | 21 | 28 | 42 | 61 | 62 |
| Group | Formulation | Vol/Dose | | | | | | | |
| A | Saline Control | 0.2 ml | X | | X | | X | X | X |
| B | AII analogue, TT carrier protein, AlOH adjuvant | 5 µg | X | | X | | X | X | X |
| C | AI analogue, TT carrier protein, AlOH adjuvant | 5 µg | X | | X | | X | X | X |
| D | AII analogue, DT carrier protein, AlOH adjuvant | 5 µg | X | | X | | X | X | X |
| E | AII analogue, KLH carrier protein, AlOH adjuvant | 5 µg | X | | X | | X | X | X |
| F | AII analogue, TT carrier protein, DEAE adjuvant | 5 µg | X | | X | | X | X | X |
| G | equal mix of AI and AII analogues TT carrier protein, AlOH adjuvant | 2x2.5 µg | X | | X | | X | X | X |
| H | AII analogue, TT carrier protein, AlOH adjuvant | 25 µg | X | | X | | X | X | X |
| J | AI analogue, TT carrier protein, AlOH adjuvant | 25 µg | X | | X | | X | X | X |
| K | AII analogue, TT carrier protein, AlOH adjuvant | 5 µg | X | X | | X | | X | X |
| L | AI analogue, TT carrier protein, AlOH adjuvant | 5 µg | X | X | | X | | X | X |

Key:

| | |
|---|---|
| AI/AII | Peptide analogues of angiotensin hormones |
| TT | Tetanus toxoid |
| DT | non-toxic recombinant Diphtheria toxin |
| KLH | Keyhole Limpet Haemocyanin |
| DEAE | Diethylaminoethyl cellulose |
| AlOH | Aluminium hydroxide gel |

**Angiotensin analogue antibody ELISA**

ELISA plate wells (Anachem) were coated with 10μg peptide equivalents of either AI or AII conjugated to bovine serum albumin (BSA) as a carrier.

The coated wells were washed with PBS (0.2% w/v)/Tween (Sigma) and blocked with 3% Marvel before diluted sera from the vaccinated rats were incubated in their respective wells. The sera had been diluted in PBS (Sigma) over a range from 2,500-20,000 fold.

Immobilised antibodies were detected in the wells using a rabbit anti-rat IgG/horseradish peroxidase conjugate and revealed using 3,3'-5,5'-tetra-methyl benzidine with $H_2O_2$ (Sigma). The reaction was terminated after 15 min at 22°C by the addition of 10% (v/v) $H_2SO_4$ (Sigma).

Colour generated was determined by absorbance at 450nm using a Packard plate reader. The resultant absorbance readings were analysed by a statistical package (SAS Institute 1997) to determine titre.

**Statistical analysis of blood pressure changes on AI Challenge**

The maximum change in mean blood pressure and heart rate over their immediate pre-challenge values were calculated for each animal and each challenge dose. Differences between treated groups and unimmunised controls were assessed by ANOVA using Dunnett's test.

**Dose response analysis**

The main effect of immunisation was to cause a parallel shift in the blood pressure dose response of animals to AI challenge. To estimate the size of this shift, a logistic model was derived and fitted to the dose response:

$$\Delta BP = \frac{\Delta BP_{\max}}{1 + (d\ IED50)^{-\alpha}} + \varepsilon \qquad \varepsilon \text{ - } N(0,\sigma^2)$$

where $d$ is the dose of AI, $BP_{max}$ is the maximal change in blood pressure, $\alpha$ a shape parameter and $ED_{50}$ is the dose of AI giving a half maximal response. Separate $ED_{50}$ estimates were obtained for each animal. Significant differences between treatment groups and unimmunised controls were assessed by ANOVA of log-transformed $ED_{50}$ values using Dunnett's multiple comparison test.

**Results**

Table 3 summarises some of the results, showing that active immunisation caused significant shifts in the pressor dose-response to AI and marked increases in antibody titres.

Clear effects on blood pressure are demonstrated with these treatments and the maximum dose shift (8.9x the control) are seen with a conjugate containing the AI analogue and tetanus toxoid on an aluminium hydroxide adjuvant.

Table 3 also demonstrates the relationship between anti-angiotensin antibody titre and response. In general, it can be seen that there is broad agreement between treatment induced titre and mean treatment induced dose shift, but no obvious dose response between groups C and J is apparent.

Figures 2 and 3 illustrate the results for control rats (Group A) and rats immunised with a conjugate of the AI analogue and tetanus toxoid, presented on an AlOH gel adjuvant at a peptide equivalent dose of 5μg (low; Group C) and 25μg (high; Group J).

Figure 2 is a graph showing pressor effects of AI in control rats (Group A) and rats immunised with AI analogue at a dose of 5 μg (low, Group C) or 25 μg (High, Group J). The y axis shows peak change in BP (mm Hg) and the x axis shows the angiotensin I dose (pmol/rat) in control, high dose group J (25 μg) and low dose group C (5 μg) animals. Errors bars shows 95% confidence interval on mean, based on pooled within group standard deviation (n=6), shown for control group only.

Figure 3 shows recordings of mean blood pressure changes in response to AI (3, 18 and 60 pmol bolus dose) in representative animals from group A(control) and Group C (5 μg dose).

**Conclusion**

Treatment with a conjugate containing an AI analogue and tetanus toxoid on an aluminium hydroxide adjuvant gives a highly significant reduction in the pressor response to exogenous AI.

Table 3

| Treatment | Median $ED_{50}$ | Mean treatment-induced dose shift | Anti-angiotensin antibody titre, ± s.e. mean (n=6) | |
|---|---|---|---|---|
| A | 8.9 | - | 0 | 0 |
| B | 39.6 | 4.5* | 15300 ± 2100 | |
| C | 79.1 | 8.9*** | 32100 ± 7800 | |
| D | 19.6 | 2.2 | 9200 ± 2200 | |
| E | 17.6 | 2.0 | 4700 ± 600 | |
| F | 15.2 | 1.7 | 5500 ± 700 | |
| G | 24.5 | 2.8 | 8300 ± 2000 | |
| H | 38.2 | 4.3* | 12100 ± 2500 | |
| J | 74.7 | 8.4*** | 20100 ± 2300 | |
| K | 13.9 | 1.6 | 5000 ± 900 | |
| L | 43.0 | 4.8* | 26100 ± 9400 | |

Median AI bolus ($pmol.rat^{-1}$) to achieve half-maximal increase in mean blood pressure ($ED_{50}$) and corresponding anti-angiotensin antibody titres in control (group A) and immunised (groups B-L) rats. Significance probabilities adjusted for multiple comparisons by Dunnett's method (*=P<0.05, **=P<0.01, ***=P<0.001).

**Example 5:** Characterisation of antibodies produced in Example 3

Antibodies produced in Example 3 were enriched by affinity chromatography as follows:

**Materials**

1 mL HiTrap protein G affinity column (Pharmacia Biotech: 17-0404-03)
Wash buffer (WB) = PBS pH 7.2
Elution buffer (EB) = 0.1M glycine (HCL) pH 2.7
Neutralizing buffer (NB) = 1M Tris (HCL) pH 9
Storage buffer (SB) = 20% ethanol (v/v)

1. Rat sera from terminal bleeds following inoculation with each of TT-NAc-CG-angiotensin I (5 mL), angiotensin I-GC-TT (7 mL), TT-NAc-CG-angiotensin II (6 mL) or angiotensin II-GC-TT (5 mL), were clarified by centrifugation, filtered through a 1 µm PTFE disc filter then dialyzed against PBS pH 7.2. Each was then separately enriched as follows.

2. The HiTrap column was washed and equilibrated with 5 mL of WB.

3. Prepared sera (Point 1) was passed once through the HiTrap column, the waste was collected and stored at -20°C.

4. The HiTrap column was washed with 5 mL of WB to remove any remaining waste sera.

5. Immobilized antibodies were eluted using 10 mL of EB. The eluent was collected in 1 ml fractions each being immediately neutralized with 0.1 mL of NB.

6. At the end of the run the HiTrap column was washed with SB and stored at 4°C.

ELISA assays were carried out as described in Example 3 using plates coated as follows:

1) for angiotensin I and II

**Materials:**

Nunc Maxisorp ELISA plates (Life Technologies: 430341A). Human Angiotensin I (Bachem: H-1680) Human Angiotensin II (Bachem: H-1705) 0.1M carbonate buffer, pH 9.8 (0.316g $Na_2CO_3$ & 0.584g $NaHCO_3$ per 0.1L)

Other materials used were as in the ELISA method detailed in Example 3 above.

1. 100 µL of either angiotensin I or angiotensin II, depending on the specific binding event to be measured @ 0.2 mg/ml of 0.1M carbonate buffer, pH 9.8) was added to a suitable number of ELISA plate wells, and incubated for 1 hour at 22°C.

2. The ELISA plate was washed with PBS/Tween, blocked with Marvel then washed with PBS/Tween again as by the ELISA method described in Example 3 above.

3. The enriched antibodies from sera raised to Tetanus toxoid (TT) conjugates TT-NAc-CG-Ang I, Ang I-GC-TT, TT-NAc-CG-Ang II and Ang II-GC-TT, were incubated (1 hour, 22°C) in the coated wells at 2.5 µg/ml of PBS pH 7.2.

The ELISA was then completed as by the method described in Example 3 but absorbance was read at 450 nm.

2) For angiotensinogen:

The ELISA for detection of native angiotensinogen (Sigma: A-2562) was performed by the method of Example 3.

The enriched antibodies from sera raised to Tetanus Toxoid (TT) conjugates TT-NAc-CG-Ang I, Ang I-GC-TT, TT-NAc-CG-Ang II and Ang II-GC-TT were incubated (1 hour 22°C) at 0.5 µg/ml of PBS pH 7.2.

Results are shown in Figures 4, 5 and 6 which show absorbance read with ELISA plates coated with angiotensin I (Fig. 4), angiotensin II (Fig. 5) and angiotensinogen (Fig. 6) and shows that antibodies raised to each of the angiotensin derivatives conjugated to TT ie TT-NAc-CG-Ang I, Ang I-GC-TT, TT-NAc-CG-Ang II and Ang II-GC-TT recognised angiotensin I, angiotensin II and angiotensinogen.

**Example 6:** Generation of further angiotensin derivatives and immunisation studies

The following angiotensin derivative peptides 1 - 6 were synthesised according to the method of Example 1

N-acetyl-Cys-Ala-Angiotensin I (1)

N-acetyl-Cys-$(Ala)_4$-Angiotensin I (2)

N-acetyl-Cys$(Gly)_6$-Angiotensin I (3)

N-acetyl-Cys-Gly-Ala-Gly-Ala-Angiotensin I (4)

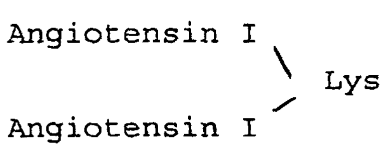

(5)

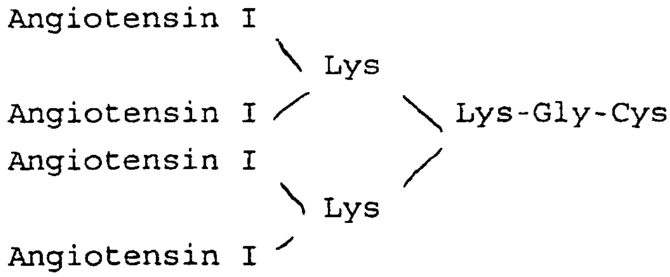


(6)

These peptides were conjugated to tetanus toxoid as described in Example 2, and formulated for immunisation studies using the protocol described in Example 3.

Antibody titres were measured using the ELISA technique described in Example 4 against the peptides used in the immunogen.

Results are shown in Figures 7 and 8 which are graphs showing, on the y axis the antibody titre, as the dilution factor to produce a SAS™ (Statistics Analysis System) designated 0.1 OD unit change, at various sample days. Each data point shown represents the mean of 5 serum samples from 5 different animals, each assayed in duplicate.

All peptides were shown to be effective in generating an anti-angiotensin I response. The responses varied in extent and duration.

Deltapak, Deltaprep, Sephadex, Alhydrogel, Tween, Marvel, Flowfusor and Millipore are registered trademarks.

## Claims

1. The use of an immunogenic angiotensin conjugate comprising at least one angiotensin I peptide coupled via a peptide carrier-binding moiety to a carrier for the manufacture of a medicament for use in combatting diseases associated with the renin-angiotensin system.

2. The use as claimed in claim 1 where said angiotensin peptide and peptide carrier binding moiety together have the sequence Asp-Arg-Val-Tyr-Ile-His-Pro-Phe-His-Leu-Gly-Cys.

3. The use as claimed in claim 1 or claim 2 wherein the carrier binding moiety contains the residue of an amino acid having a reactive side chain.

4. The use as claimed in any one of the preceding claims wherein the carrier binding moiety is a peptide extension at the N- or the C-terminus of an angiotensin peptide.

5. The use as claimed in any one of the preceding claims wherein the carrier binding moiety is a peptide extension at the N-terminus of an angiotensin peptide.

6. The use as claimed in any one of the preceding claims wherein the carrier is a polypeptide.

7. The use as claimed in any one of the preceding claims wherein the carrier is selected from the purified protein derivative of tuberculin, tetanus toxoid, diphtheria toxoid, keyhole limpet haemocyanin or derivatives thereof.

8. The use as claimed in any one of the preceding claims wherein said conjugate elicits a cross-reactive immune response with angiotensin I, angiotensin II, and/or angiotensinogen molecules.

9. The use as claimed in any one of the preceding claims wherein said disease is congestive heart failure or hypertension.

10. The use as claimed in any one of claims 1 to 8 for the modulation of blood pressure.

## Patentansprüche

1. Die Verwendung eines immunogenen Angiotensin-Konjugats, das mindestens ein Angiotension-I-Peptid umfasst, das über einen Peptid-Träger-bindenden Teil an einen Träger gekoppelt ist, zur Herstellung eines Medikamentes zur Verwendung bei Bekämpfung von Krankheiten, die mit dem Renin-Angiotensinsystem verknüpft sind.

2. Die Verwendung nach Anspruch 1, wobei das Angiotensin-Peptid und der Peptid-Träger-bindende Teil zusammen die Sequenz Asp-Arg-val-Tyr-Ile-His-Pro-Phe-His-Leu-Gly-Cys besitzen.

3. Die Verwendung nach Anspruch 1 oder 2, worin der Träger-bindende Teil den Rest einer eine reaktive Seitenkette aufweisenden Aminosäure beinhaltet.

4. Die Verwendung nach einem der vorhergehenden Ansprüche, worin der Träger-bindende Teil einer Peptiderweiterung an dem N- oder dem C-Ende eines Angiotensin-Peptids entspricht.

5. Die Verwendung nach einem der vorhergehenden Ansprüche, worin der Träger-bindende Teil einer Peptiderweiterung an dem N-Ende eines Angiotensin-Peptids entspricht.

6. Die Verwendung nach einem der vorhergehenden Ansprüche, worin der Träger ein Polypeptid ist.

7. Die verwendung nach einem der vorhergehenden Ansprüche, worin der Träger ausgewählt ist aus den gereinigten Proteinderivaten von Tuberculin, Tetanustoxoid, Diphtherietoxoid, Keyhole limpet haemocyanin oder aus Derivaten davon.

8. Verwendung nach einem der vorhergehenden Ansprüche, worin das Konjugat eine kreuzreaktive Immunreaktion mit Angiotensin I, Angiotensin II, und/oder angiotensinogenen Molekülen auslöst.

9. Verwendung nach einem der vorhergehenden Ansprüche, worin die Krankheit kongestive Herzinsuffizienz oder Bluthochdruck ist.

10. Verwendung nach einem der Ansprüche 1 bis 8 zur Modulation des Blutdrucks.

## Revendications

1. Utilisation d'un conjugué d'angiotensine immunogène comprenant au moins un peptide d'angiotensine 1 couplé par l'intermédiaire d'un fragment peptidique de liaison à un support, à un support pour la fabrication d'un médicament pour utilisation dans la lutte contre les maladies associées au système rénine-angiotensine.

2. Utilisation selon la revendication 1 dans laquelle lesdits peptide d'angiotensine et fragment peptidique de liaison à un support ont conjointement la séquence Asp-Arg-Val-Tyr-Ue-His-Pro-Phe-His-Leu-Gly-Cys.

3. Utilisation selon la revendication 1 ou la revendication 2 dans laquelle le fragment de liaison à un support contient le résidu d'un acide aminé ayant une chaîne latérale réactive.

4. Utilisation selon l'une quelconque des revendications précédentes dans laquelle le fragment de liaison à un support est une extension peptidique à l'extrémité N ou C d'un peptide d'angiotensine.

5. Utilisation selon l'une quelconque des revendications précédentes dans laquelle le fragment de liaison à un support est une extension peptidique à l'extrémité N d'un peptide d'angiotensine.

6. Utilisation selon l'une quelconque des revendications précédentes dans laquelle le support est un polypeptide.

7. Utilisation selon l'une quelconque des revendications précédentes dans laquelle le support est choisi parmi le dérivé de protéine purifié de la tuberculine, l'anatoxine du tétanos, l'anatoxine de la diphtérie, l'hémocyanine de patelle ou leurs dérivés.

8. Utilisation selon l'une quelconque des revendications précédentes dans laquelle ledit conjugué provoque une

réponse immunitaire croisée avec l'angiotensine I, l'angiotensine II et/ou les molécules angiotensinogènes.

**9.** Utilisation selon l'une quelconque des revendications précédentes dans laquelle ladite maladie est l'insuffisance cardiaque congestive ou l'hypertension.

**10.** Utilisation selon l'une quelconque des revendications 1 à 8 pour la modulation de la pression artérielle.

FIG. 1.

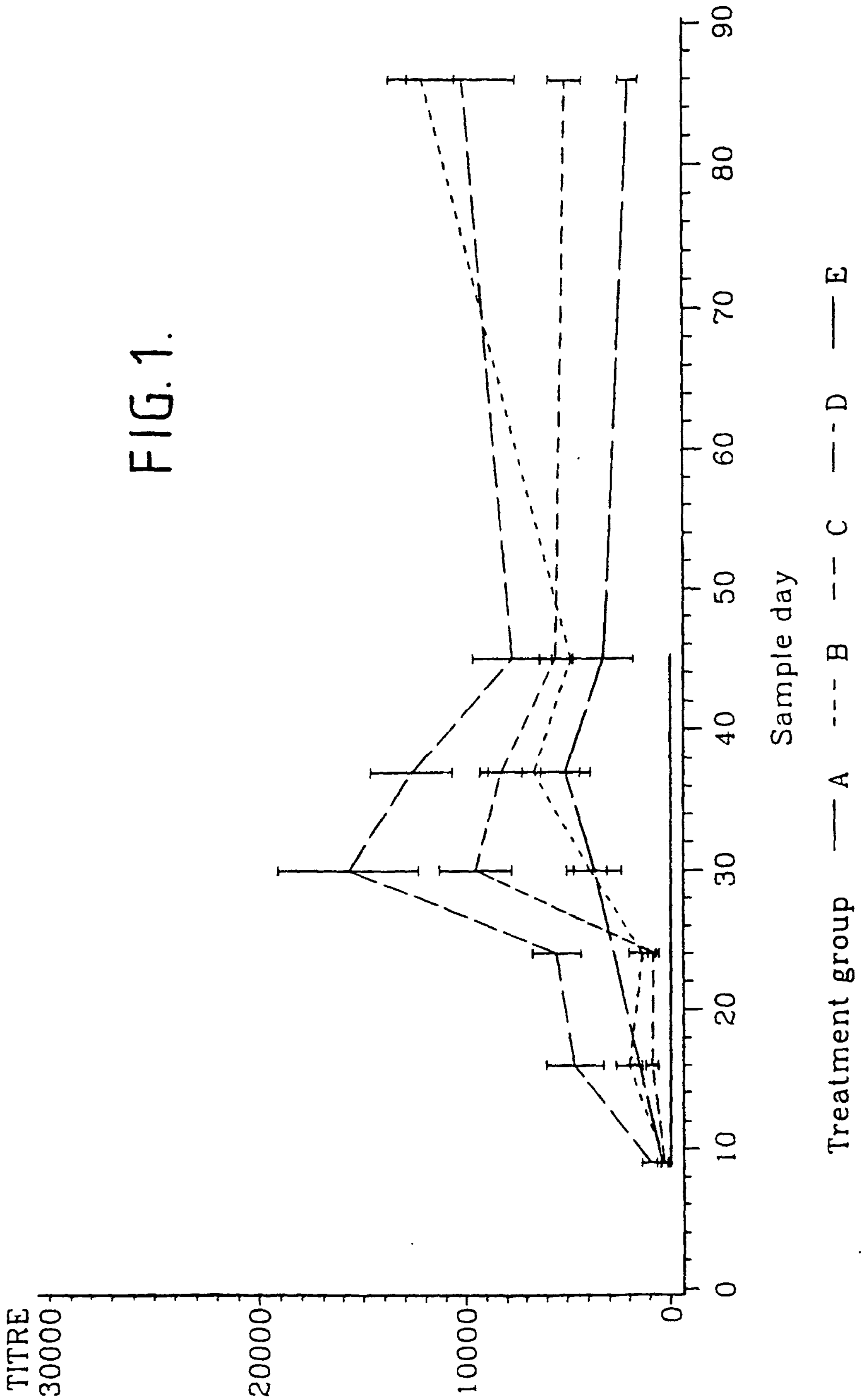
TITRE
30000
20000
10000
0
0
10
20
30
40
50
60
70
80
90
Sample day
Treatment group — A --- B -- C — - D —— E

FIG. 2.

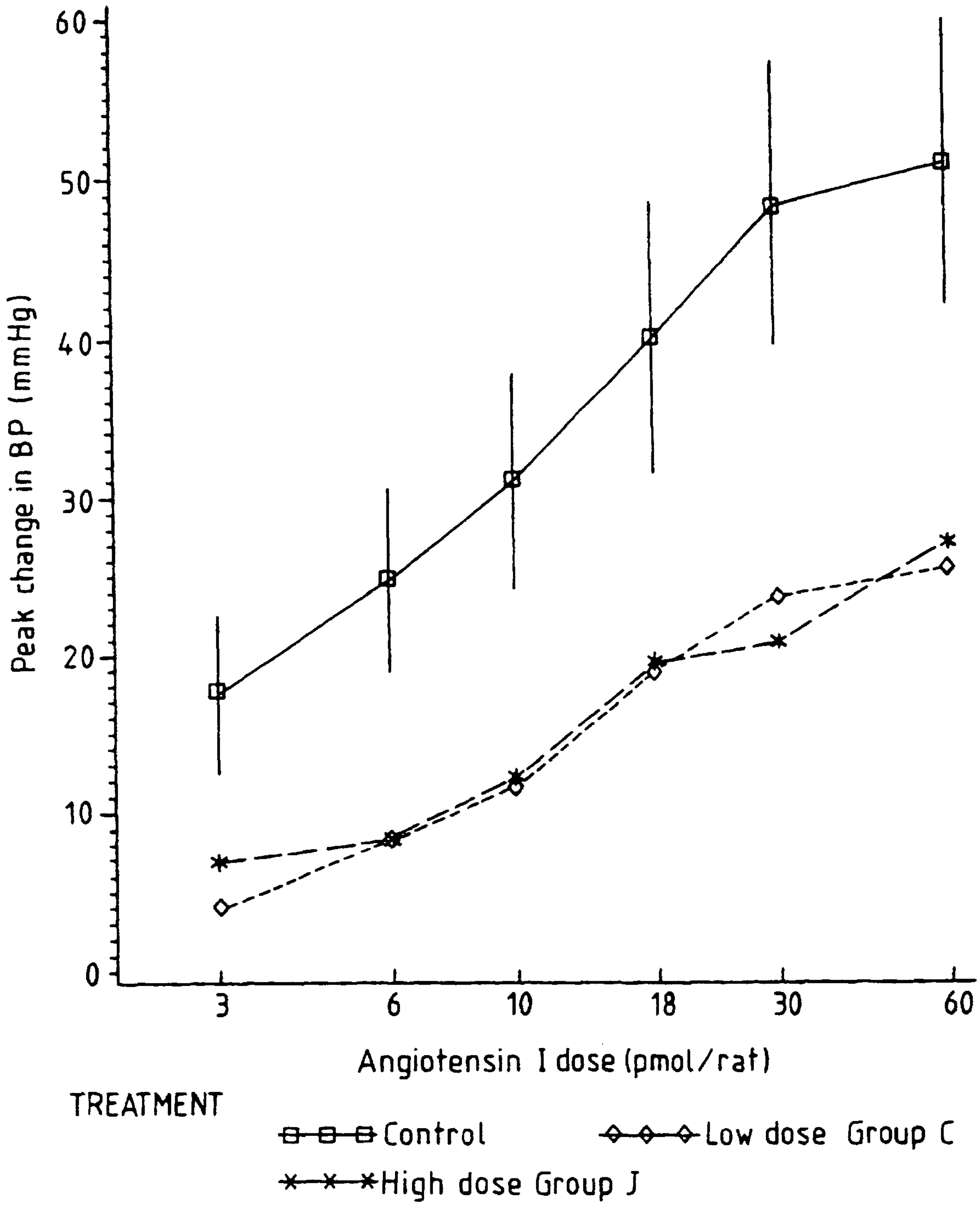
Peak change in BP (mmHg)
60
50
40
30
20
10
0
3
6
10
18
30
60
Angiotensin I dose (pmol/rat)
TREATMENT
Control
Low dose Group C
High dose Group J

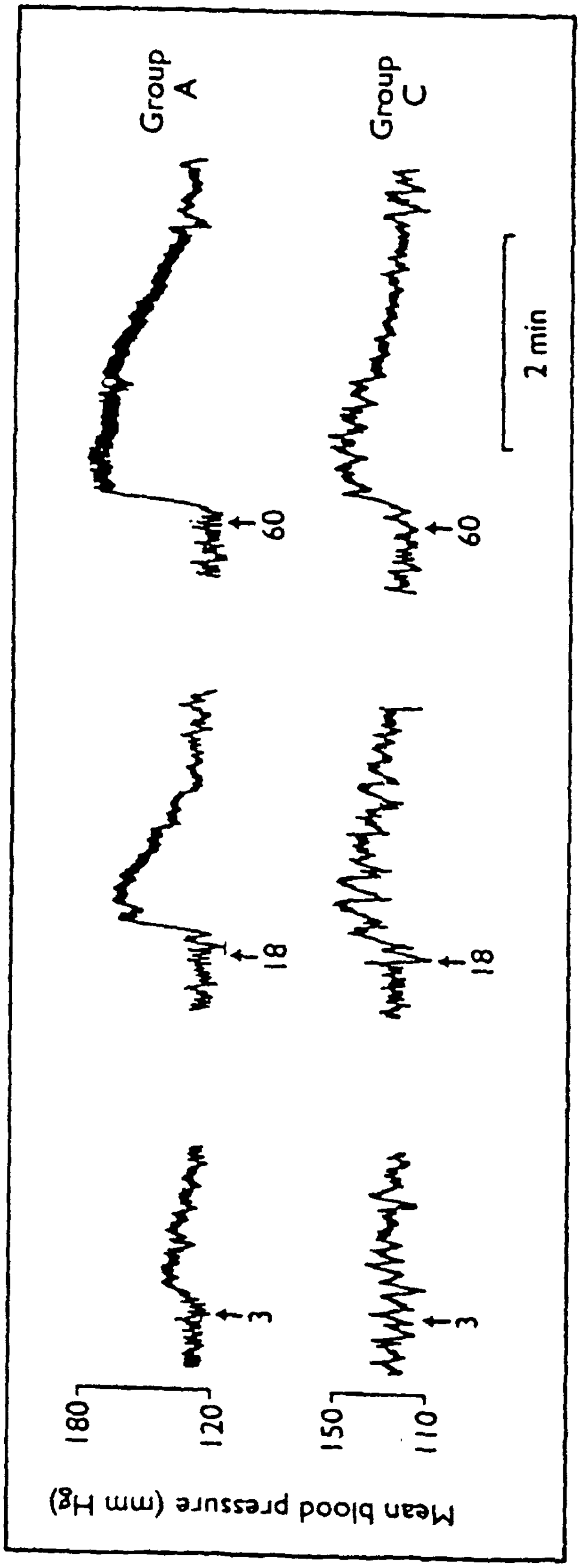
Mean blood pressure (mm Hg)
180
120
150
110
3
18
60
Group A
Group C
2 min

FIG. 3.

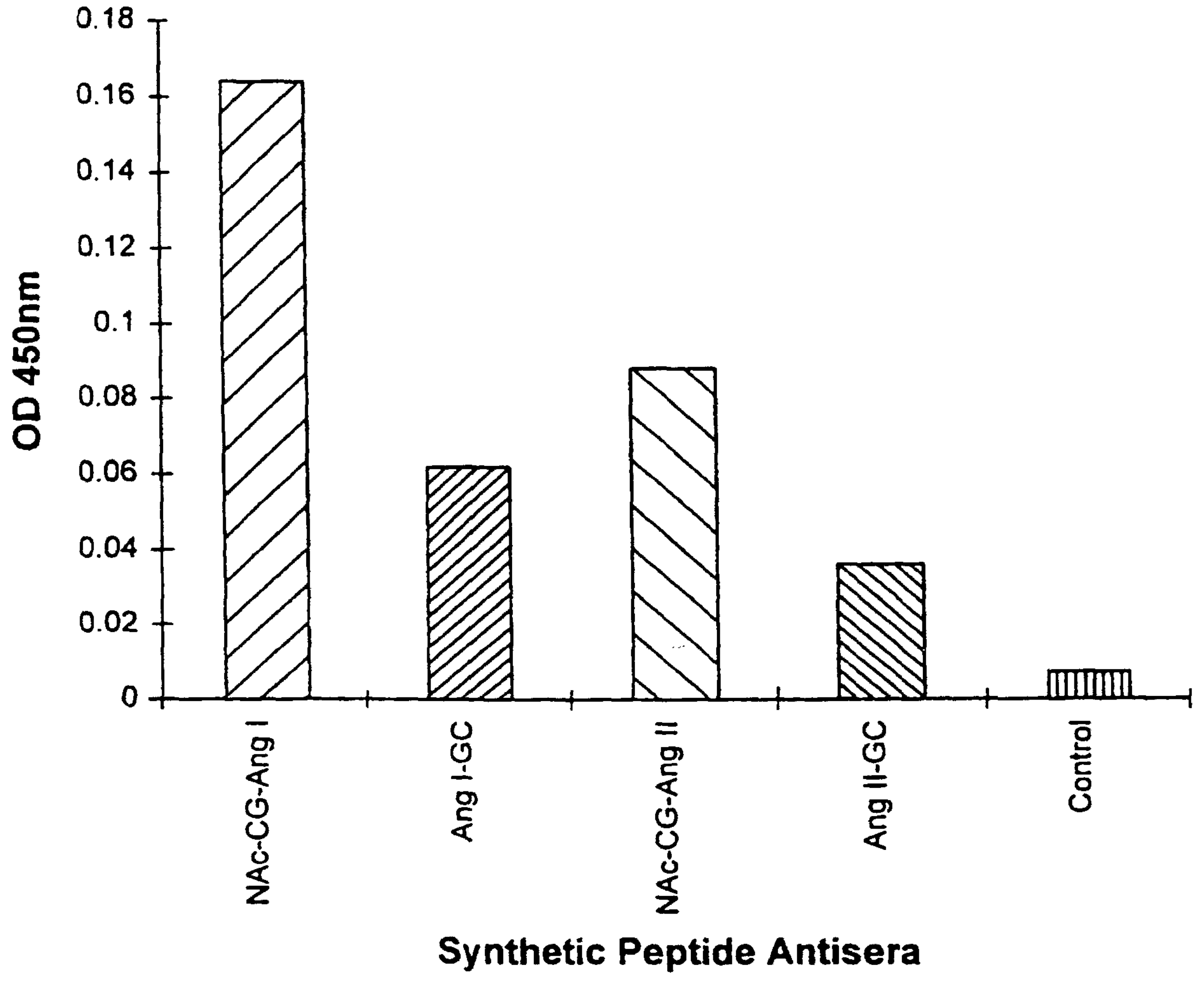
0.18
0.16
0.14
0.12
0.1
0.08
0.06
0.04
0.02
0
OD 450nm
NAc-CG-Ang I
Ang I-GC
NAc-CG-Ang II
Ang II-GC
Control
Synthetic Peptide Antisera

FIG. 4.

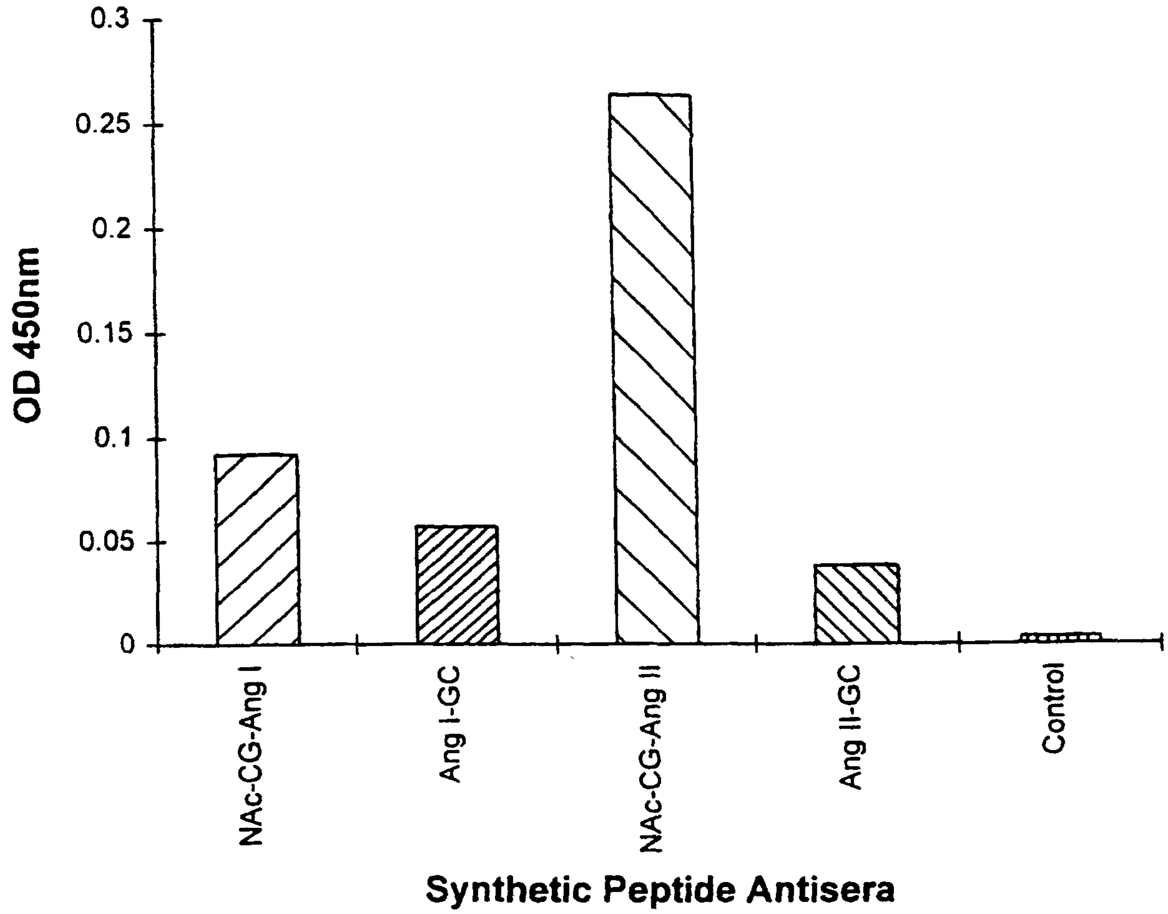
0.3
0.25
0.2
0.15
0.1
0.05
0
OD 450nm
NAc-CG-Ang I
Ang I-GC
NAc-CG-Ang II
Ang II-GC
Control
Synthetic Peptide Antisera

FIG. 5.

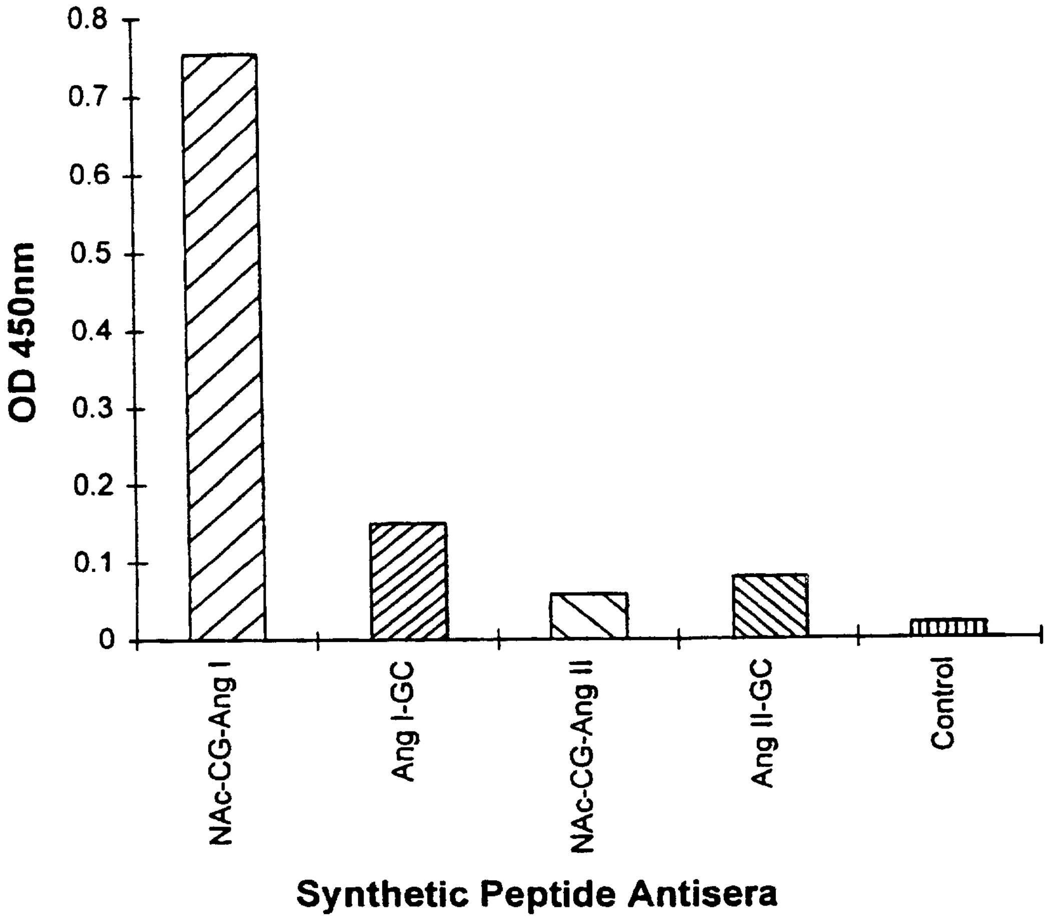
0.8
0.7
0.6
0.5
0.4
0.3
0.2
0.1
0
OD 450nm
NAc-CG-Ang I
Ang I-GC
NAc-CG-Ang II
Ang II-GC
Control
Synthetic Peptide Antisera

FIG. 6.

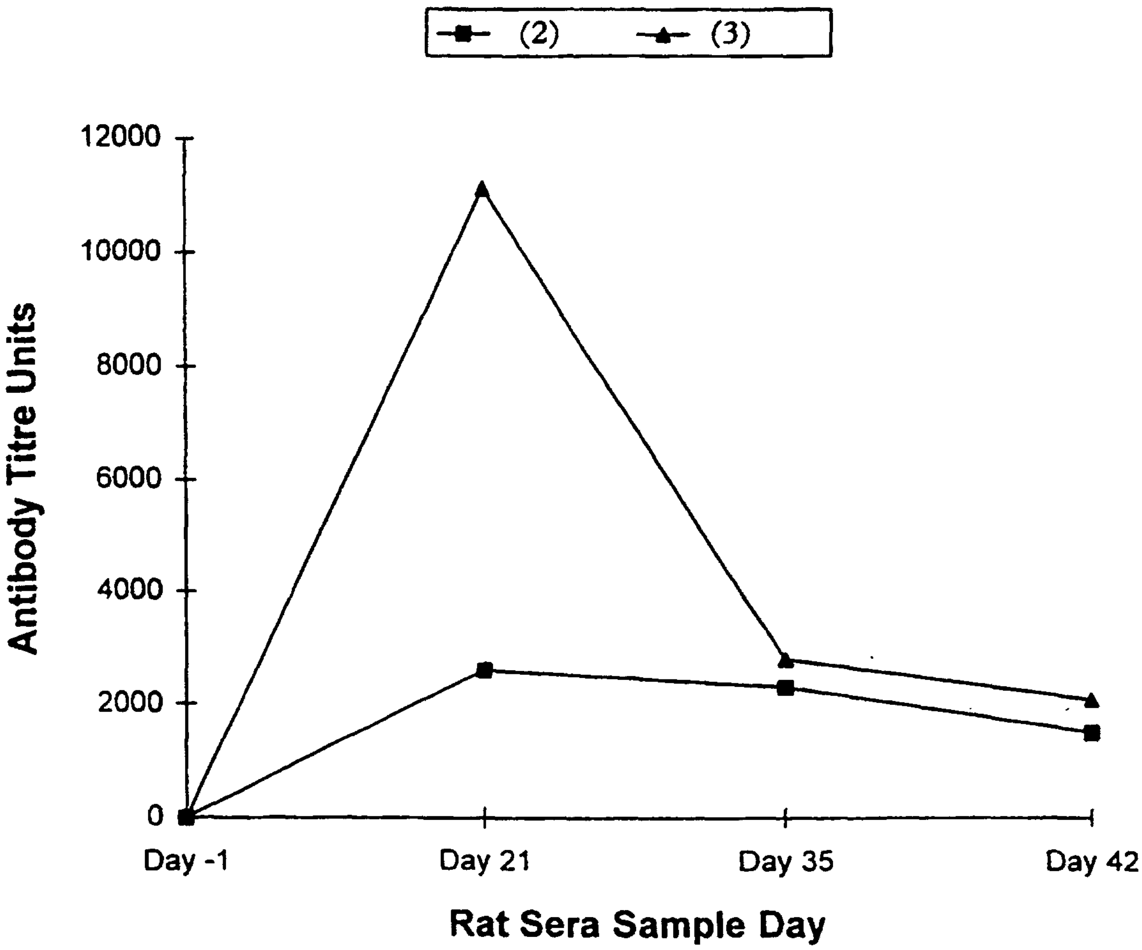
(2)
(3)
12000
10000
8000
6000
4000
2000
0
Antibody Titre Units
Day -1
Day 21
Day 35
Day 42
Rat Sera Sample Day

FIG. 7.

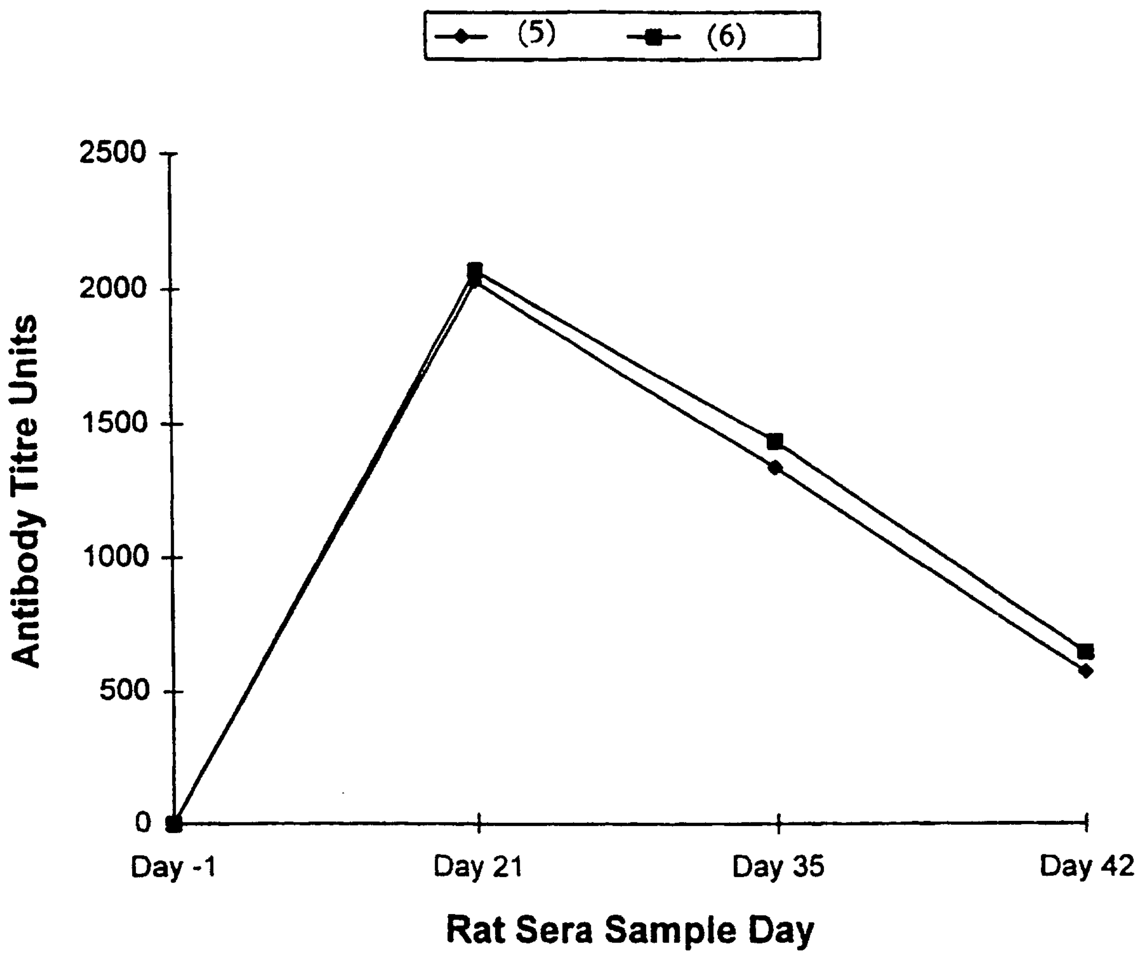
(5)
(6)
2500
2000
1500
1000
500
0
Antibody Titre Units
Day -1
Day 21
Day 35
Day 42
Rat Sera Sample Day

FIG. 8.